# EUROPEAN PATENT APPLICATION

(11) **EP 2 255 848 A2**
(43) Date of publication of application: **01.12.2010**
(21) Application number: 10176175.7
(22) Date of filing: 04.09.2007
(51) Int. Cl.: A61P 25/28, A61P 25/26, A61K 45/06, A61K 31/4245, A61K 31/4439

(54) **Pharmaceutical combinations of a nicotine receptor modulator and a cognitive enhancer**

(30) Priority: 04.09.2006 DK 200601139; 06.09.2006 US 824622 P
(62) Divisional of application: 07803198.6
(71) Applicant: NeuroSearch AS, 2750 Ballerup (DK)
(72) Inventor: Olsen, Gunnar, M., 2765, Smørum (DK); Peters, Dan, 2750, Ballerup (DK); Dahl, Bjarne, H., 3540, Lynge (DK); Christensen, Jeppe, Kejser, 2750, Ballerup (DK); Loechel, Steven, Charles, 2000, Frederiksberg (DK); Timmermann, Daniel, B., 2750, Ballerup (DK)
(74) Representative: Abildgren, Michael Padkjaer

(57) **Abstract**

This invention relates to novel pharmaceutical compositions comprising therapeutically effective combination of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug. The pharmaceutical compositions for use according to the invention are contemplated particularly useful for combating cognitive disorders.

## Description

### TECHNICAL FIELD

This invention relates to novel pharmaceutical compositions comprising therapeutically effective combination of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug. The pharmaceutical compositions for use according to the invention are contemplated particularly useful for combating cognitive disorders.

### BACKGROUND ART

Cognitive deficit is one or more malfunction(s) in high level information processing carried out by the human brain. This includes sensory information, attention, perception, consolidation of information, recall of information, reconstruction, calculation ability, and executive functions such as planning, problem-solving, self-monitoring and use of speech.

Cognitive deficits are part of the clinical picture in Depression, ADHD, Schizophrenia, Parkinson's disease, age associated memory impairment, dementia of Alzheimer type and Alzheimer's disease.

### SUMMARY OF THE INVENTION

Investigations carried out by the inventors have lead to the conclusion that combinations of a positive allosteric modulator of nicotine receptors and a cognitive enhancer constitute a particularly useful combination for use in therapy associated with cognitive deficits.

In its first aspect the invention provides pharmaceutical compositions comprising a therapeutically effective amount of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; or pharmaceutically-acceptable addition salts thereof, together with one or more adjuvants, excipients, carriers and/or diluents.

In another aspect the invention relates to the use of a combination of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; or pharmaceutically-acceptable addition salts thereof, for the manufacture of a medicament for the treatment, prevention or alleviation of a cognitive dysfunction of a mammal, including a human.

In a third aspect the invention provides a kit of parts comprising at least two separate unit dosage forms (A) and (B), wherein (A) comprises a positive allosteric modulator of nicotine receptors; and (B) comprises a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; and optionally (C) instructions for the simultaneous, sequential or separate administration of the positive allosteric nicotine receptor modulator of (A) and the cognitive enhancer of (B) to a patient in need thereof.

In a final aspect the invention provides a method of treatment, prevention or alleviation of a cognitive dysfunction of a living animal body, including a human, which method comprises the step of administering to such a living animal body in need thereof, a therapeutically effective amount of a combination of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; or pharmaceutically-acceptable addition salts thereof.

Other objects of the invention will be apparent to the person skilled in the art from the following detailed description and examples.

### DETAILED DISCLOSURE OF THE INVENTION

In its first aspect the invention provides pharmaceutical compositions comprising a combination of therapeutically effective amounts of a positive allosteric modulator of nicotine receptors and a cognitive enhancer.

### Positive Allosteric Nicotine Receptor Modulators

Only a few positive allosteric nicotine receptor modulators are reported in the art, incl. N-(5-chloro-2,4-dimethoxyphenyl)-N'-(5-methyl-isoxazol-3-yl-urea (PNU-120596), described in e.g. WO 2003/093250, and Galantamine (Razadyne™, Reminyl™).

The positive allosteric nicotine receptor modulator for use according to the invention may be selected from any drug or drug candidate available or described in the art, and in particular selected from those described in more details herein.

### Oxadiazole Derivatives

The positive allosteric nicotine receptor modulator for use according to the invention may in particular be an oxadiazole derivative selected from the group consisting of
3-Cyclopropyl-5-(5-nitro-furan-2-yl)-[1,2,4]oxadiazole;
5-(5-Nitro-furan-2-yl)-3-phenyl-[1,2,4]oxadiazole;
5-(5-Nitro-furan-2-yl)-3-(4-fluoro)-phenyl-[1,2,4]oxadiazole;
5-(5-Nitro-furan-2-yl)-3-benzyl-[1,2,4]oxadiazole;
5-(5-Nitro-furan-2-yl)-3-thiophen-2-yl-[1,2,4]oxadiazole;
2-(5-(5-Nitro-furan-3-yl)-[1,2,4]oxadiazol-3-yl)-pyridine;
3-(5-(3-Nitro-phenyl)-[1,2,4]oxadiazol-3-yl)-pyridine;
3-(5-Furan-2-yl-[1,2,4]oxadiazol-3-yl)-pyridine;
3-(5-(5-Nitro-furan-3-yl)-[1,2,4]oxadiazol-3-yl)-pyridine;
3-(5-Furan-3-yl-[1,2,4]oxadiazol-3-yl)-pyridine;
3-[5-(1H-Pyrrol-2-yl)-[1,2,4]oxadiazol-3-yl]-pyridine;
4-(5-Furan-2-yl-[1,2,4]oxadiazol-3-yl)-pyridine;
2-[5-(5-Nitro-furan-2-yl)-[1,2,4]oxadiazol-3-yl]-pyrazine;
3-[5-(1-Methyl-1H-pyrrol-2-yl)-[1,2,4]oxadiazol-3-yl]-pyridine;
3-[5-(1H-Pyrazol-4-yl)-[1,2,4]oxadiazol-3-yl]-pyridine;
3-[5-(2-Methyl-thiazol-4-yl)-[1,2,4]oxadiazol-3-yl]-pyridine;
3-[5-(4-Nitro-phenyl)-[1,2,4]oxadiazol-3-yl]-pyridine;
2-[5-(3-Nitro-phenyl)-[1,2,4]oxadiazol-3-yl]-pyridine;
3-(5-Phenyl-[1,2,4]oxadiazol-3-yl)-pyridine;
3-(3-Pyridin-3-yl-[1,2,4]oxadiazol-5-yl)-benzonitrile;
3-[5-(3-Chloro-phenyl)-[1,2,4]oxadiazol-3-yl]-pyridine;
3-Phenyl-5-(thiophen-3-yl)-[1,2,4]oxadiazole;
4-[5-(3-Nitro-phenyl)-[1,2,4]oxadiazol-3-yl]-pyridine;
3-[5-(3-Fluoro-phenyl)-[1,2,4]oxadiazol-3-yl]-pyridine;
2-[5-(3-Nitro-phenyl)-[1,2,4]oxadiazol-3-yl]-pyrazine;
3-Phenyl-5-(thiophen-2-yl)-[1,2,4]oxadiazole;
3-[5-(2-Nitro-phenyl)-[1,2,4]oxadiazol-3-yl]-pyridine;
3-[5-(3-Trifluoromethyl-phenyl)-[1,2,4]oxadiazol-3-yl]-pyridine;
3-[3-(3-Nitro-phenyl)-[1,2,4]oxadiazol-5-yl]-pyridine;
6-(Pyridin-3-yl-[1,2,4]oxadiazol-5-yl)-pyridine-2-carbonitrile;
5-(3-Pyridin-3-yl-[1,2,4]oxadiazol-5-yl)-furan-2-carbonitrile;
5-(3-Pyridin-3-yl-[1.2.4]oxadiazol-5-yl)-thiophene-2-carbonitrile; and 3-(3-Pyridin-3-yl-[1,2,4]oxadiazol-5-yl)-phenylamine;
any of its isomers or any mixture of isomers, or a pharmaceutically-acceptable addition salt thereof.

In a most preferred embodiment the oxadiazole derivative for use according to the invention is
3-(3-Pyridin-3-yl-[1,2,4]oxadiazol-5-yl)-benzonitrile; or
5-(3-Pyridin-3-yl-[1.2.4]oxadiazol-5-yl)-thiophene-2-carbonitrile;
PNU-120596; or
Galantamine;
any of its isomers or any mixture of isomers, or a pharmaceutically-acceptable addition salt thereof.

### Pharmaceutically Acceptable Salts

The oxadiazole derivative of the invention may be provided in any form suitable for the intended administration. Suitable forms include pharmaceutically (i.e. physiologically) acceptable salts, and pre- or prodrug forms of the compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a compound of the invention include alkali metal salts, such as the sodium salt of a compound of the invention containing a carboxy group.

### Methods of Producing Oxadiazole Derivatives

The oxadiazole derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Cognition Enhancers

While the positive allosteric nicotine receptor modulators for use according to the invention are described above, the cognition enhancers for use according to the invention may be selected from the group consisting of a nicotine acetylcholine receptor agonist, an acetylcholine esterase inhibitor, a positive AMPA receptor modulator, an antipsychotic drug, an antidepressant drug and an anti Parkinson drug, as described in more details below.

### Nicotine Acetylcholine Receptor Agonists

The nicotine acetylcholine receptor agonists for use according to the invention are known in the art and may be commercially available or may be obtained as described in the literature.
Nicotine is commercially available from e.g. Sigma-Alldrich.
ABT-594 is described in e.g. WO 98/25920.
SSR-180711A is described in e.g. WO 00/58311 or EP 1231212.
PNU-282987 is described in e.g. EP 99789.
AR-R17779 is described in e.g. WO 96/06098.

Varenicline is available from Pfizer (Chantix™) and described in e.g. WO 99/35131.

Isopronicline (TC-1734) is available from Targacept and described in e.g. WO 99/65876 and WO 2000/075110.

In another preferred embodiment the nicotine acetylcholine receptor agonists for use according to the invention are N-substituted diazabicyclic compounds described in e.g. WO 2001/81347, WO 2004/106342, WO 2006/019660 or WO 2006/019668.

In a third preferred embodiment the nicotine acetylcholine receptor agonists for use according to the invention are diazabicyclic compounds described in e.g. WO 2001/081347.

In a fourth preferred embodiment the nicotine acetylcholine receptor agonists for use according to the invention are piperazine or homopiperazine derivatives described in e.g. WO 99/21834.

In a most preferred embodiment the nicotine acetylcholine receptor agonists for use according to the invention is
1-(3-Pyridyl)-homopiperazine; or
1-(3-Chloro-5-pyridyl)homopiperazine,
any of its isomers or any mixture of isomers, or a pharmaceutically-acceptable addition salt thereof.

### Acetylcholine Esterase Inhibitors

The acetylcholine esterase inhibitors for use according to the invention are known in the art may be commercially available under different brand names, e.g.
Tacrin (Cognex™),
Donepezil (Aricept™),
Rivastigmine (Exelon™), and
Galantamine (Reminyl™).

### Positive AMPA Receptor Modulators

The positive AMPA receptor modulators for use according to the invention are known in the art and may be commercially available under different brand names, or may be obtained as described in the literature.

CX-516 (Ampalex™) and CX-546, available from Cortex Pharmaceuticals, Inc. and described in e.g. WO 94/02475 or WO 97/07799.
CX-614 is described in e.g. WO 97/36907.
Others may be commercially available under different brand names, e.g.
Cyclothiazid (commercially available from e.g. Sigma-Alldrich),
Piracetam (Nootropyl™), and
Aniracetam (Draganon™, Sarpul™, Ampamet™, Reset™).

### Antipsychotics

The antipsychotic drugs for use according to the invention are known in the art and may be commercially available under different brand names, e.g.
the classical dopamine antagonists, in particular Haloperidol (Haldol™), Fluphenazine (Prolixin™), Chlorpromazine (Largactil™, Thorazine™), and Pimozide (Orap™); and
the atypical dopamine antagonists, in particular Clozapine (Clozaril™), Olanzapine (Zyprexa™), Ziprasidone (Geodon™), Risperidone (Risperdal™), Quetiapine (Seroquel™), Aripiprazole (Abilify™), Sertindole (Serlect™, Serdolect™), Zotepine (Nipolept™) and Amisulpride (Solian™).

### Antidepressant Drugs

The an antidepressant drug for use according to the invention are known in the art and include the selective dopamine, noradrenaline or serotonin reuptake inhibitors, as well as the mixed monoamine uptake inhibitors.

The antidepressant drug for use according to the invention may be commercially available under different brand names, e.g. Bupropion (Wellbutrin™), Citalopram (Cipramil™), Desipramine (Norpramin™, Pertofrane™), Duloxetine (Cymbalta™, Xeristar™, Yentreve™), Escitalopram (Celexa™, Lexapro™), Fenfluramine (Pondimin™), Fluoxetine (Prozac™), Fluvoxamine (Luvox™), Imipramine (Tofranil™), Mirtazapine (Remeron), Paroxetine (Seroxat™, Paxil™), Radafaxine, Sibutramine (Meridia™), Sertraline (Zoloft™) and Venlafaxine (Efexor™).

Other compounds for use as antidepressant drugs according to the invention are those described in e.g. WO 97/30997, and in particular
2-methoxymethyl-3-(3,4-dichlorophenyl)-tropane;
2-isopropoxymethyl-3-(3,4-dichlorophenyl)-tropane;
2-ethoxymethyl-3-(3,4-dichlorophenyl)-tropane;
2-cyclopropylmethyloxymethyl-3-(3,4-dichlorophenyl)-tropane;
2-methoxymethyl-3-(4-chlorophenyl)-tropane;
N-Normethyl-2-methoxymethyl-3-(4-chlorophenyl)-tropane;
2-ethoxymethyl-3-(4-chlorophenyl)-tropane;
N-normethyl-2-methoxymethyl-3-(3,4-dichlorophenyl)-tropane;
N-normethyl-2-ethoxymethyl-3-(3,4-dichlorophenyl)-tropane;
N-Normethyl-2-ethoxymethyl-3-(4-chlorophenyl)-tropane;
2-ethylthiomethyl-3-(3,4-dichlorophenyl)-tropane;
2-cyclopropylmethyloxymethyl-3-(4-chlorophenyl)-tropane; and N-normethyl-2-cyclopropylmethyloxymethyl-3-(4-chlorophenyl)-tropane;
any of its isomers or any mixture of isomers, or a pharmaceutically-acceptable addition salt thereof.

Most preferred antidepressant drugs for use according to the invention are Duloxetine, Escitalopram, Tesofensine and Venlafaxine.

### Anti Parkinson Drugs

The anti Parkinson drugs for use according to the invention are known in the art may be commercially available under different brand names, e.g.
Nomifensine (Merital™),
Bromocriptine (Parlodel™),
Levodopa (Dopar™, Larodopa™),
Pergolide (Permax™),
Pramipexole (Mirapex™), and
Ropinerole (Requip™).

### Biological Activity

The pharmaceutical compositions for use according to the invention are contemplated particularly useful for combating cognitive disorders.

In a preferred embodiment the cognitive disorder contemplated according to the invention is learning deficit, memory deficit, memory dysfunction, memory impairment associated with depresssion or anxiety, cognitive or attention deficits related to schizophrenia, Alzheimer's Disease (AD), mild cognitive impairment, age-related cognitive decline, vascular dementia, Parkinson's disease, Huntington's disease, schizophrenia, Down's syndrome, stroke, traumatic brain injury (TBI), AIDS associated dementia or attention deficit disorder.

In another preferred embodiment the cognitive disorder contemplated according to the invention is learning deficit, attention deficit, memory deficits and dysfunction, cognitive or attention deficits related to schizophrenia, Alzheimer's Disease (AD), mild cognitive impairment or age-related cognitive decline.

In a most preferred embodiment the cognitive disorders contemplated according to the invention are cognitive deficits following depression, ADHD, Schizophrenia or Parkinson's disease; age associated memory impairment; and dementia of Alzheimer type and Alzheimer's disease.

### Pharmaceutical Compositions

In another aspect the invention provides novel pharmaceutical compositions comprising a therapeutically effective amount of oxadiazole derivative of the invention.

While a compound of the invention for use in therapy may be administered in the form of the raw compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the oxadiazole derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by the skilled person by use of standard methods and conventional techniques appropriate to the desired formulation. When desired, compositions adapted to give sustained release of the active ingredient may be employed.

In a preferred embodiment, when the pharmaceutical composition of the invention is intended for treating patients with withdrawal symptoms caused by nicotine addiction, formulations such as gums, patches, sprays, inhalers, aerosols, etc., are contemplated.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### Pharmaceutical Kits of Parts

According to the invention there is also provided a kit of parts comprising at least two separate unit dosage forms (A) and (B):
(A) a positive allosteric modulator of nicotine receptors; and
(B) a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; and optionally
(C) instructions for the simultaneous, sequential or separate administration of the positive allosteric nicotine receptor modulator of (A) and the cognitive enhancer of (B) to a patient in need thereof.
The positive allosteric nicotine receptor modulators for use according to the invention and the cognitive enhancer for use according to the invention may preferably be provided in a form that is suitable for administration in conjunction with the other. This is intended to include instances where one or the other of two formulations may be administered (optionally repeatedly) prior to, after, and/or at the same time as administration with the other component.

Also, the positive allosteric nicotine receptor modulators for use according to the invention and the cognitive enhancer for use according to the invention may be administered in a combined form, or separately or separately and sequentially, wherein the sequential administration is close in time or remote in time. This may in particular include that two formulations are administered (optionally repeatedly) sufficiently closely in time for there to be a beneficial effect for the patient, that is greater over the course of the treatment of the relevant condition than if either of the two formulations are administered (optionally repeatedly) alone, in the absence of the other formulation, over the same course of treatment. Determination of whether a combination provides a greater beneficial effect in respect of, and over the course of treatment of, a particular condition, will depend upon the condition to be treated or prevented, but may be achieved routinely by the person skilled in the art.

When used in this context, the terms "administered simultaneously" and "administered at the same time as" include that individual doses of the positive allosteric nicotine receptor modulator and the cognitive enhancer are administered within 48 hours, e.g. 24 hours, of each other.

Bringing the two components into association with each other, includes that components (A) and (B) may be provided as separate formulations (i.e. independently of one another), which are subsequently brought together for use in conjunction with each other in combination therapy; or packaged and presented together as separate components of a "combination pack" for use in conjunction with each other in combination therapy.

### Methods of Therapy

In another aspect the invention provides methods of treatment, prevention or alleviation of a cognitive dysfunction of a living animal body, including a human, which method comprises the step of administering to such a living animal body in need thereof, a therapeutically effective amount of a combination of a positive allosteric modulator of nicotine receptors; and a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; or pharmaceutically-acceptable addition salts thereof.

The preferred indications contemplated according to the invention are those stated above.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

A satisfactory result can, in certain instances, be obtained at a dosage as low as 0.005 mg/kg i.v. and 0.01 mg/kg p.o. The upper limit of the dosage range is about 10 mg/kg i.v. and 100 mg/kg p.o. Preferred ranges are from about 0.001 to about 1 mg/kg i.v. and from about 0.1 to about 10 mg/kg p.o.

### BRIEF DESCRIPTION OF THE DRAWINGS

The present invention is further illustrated by reference to the accompanying drawing, in which:
Fig. 1 shows the (-)nicotine concentration-response curve recorded in the presence of 1 µM of 3-(3-Pyridin-3-yl-[1,2,4]oxadiazol-5-yl)-benzonitrile (Compound 4.15) as positive allosteric modulator [▼= Compound 4.15 + nicotine; ■ = nicotine];
Fig. 2 shows mouse marble burying 15 minutes after s.c. dosing of 1-(3-Pyridyl)-homopiperazine (Compound 1 F of WO 99/21834) as cognition enhancer, and ED₅₀ was determined 0.56 mg/kg; and
Figs. 3A-3D show mouse marble burying 15 minutes after s.c. dosing of 1-(3-Pyridyl)-homopiperazine (Compound 1 F of WO 99/21834) as cognition enhancer, and 30 minutes after p.o. dosing of 3-(3-Pyridin-3-yl-[1,2,4]oxadiazol-5-yl)-benzonitrile (Compound 4.15) as positive allosteric modulator:

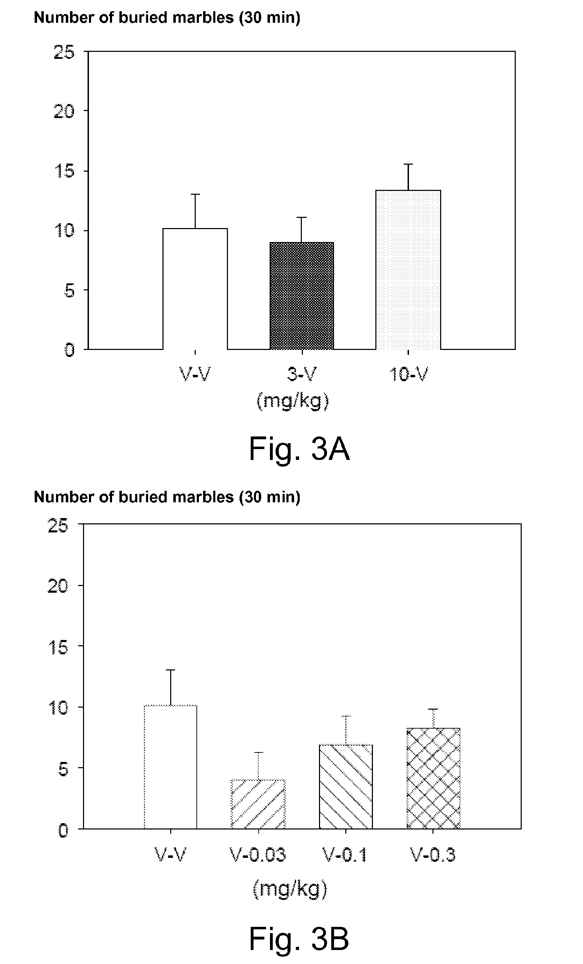

Fig. 3A shows no effect of Compound 4.15 alone (dosed p.o. at 3 and 10 mg/kg);
Fig. 3B shows no effect of Compound 1 F alone (dosed s.c. at 0.03, 0.1 and 0.3 mg/kg); and

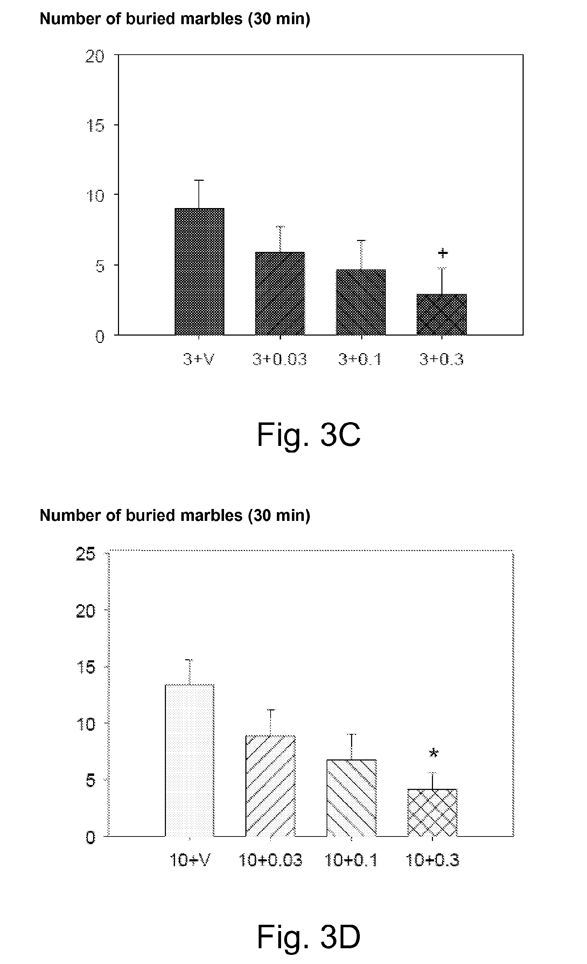

Figs. 3C and 3D show significant synergistic effect of using a combination of Compound F (dosed s.c. at 0.3 mg/kg) and Compound 4.15 (dosed p.o. at 3 and 10 mg/kg), and ED₅₀ was determined 0.1 mg/kg.

### EXAMPLES

The invention is further illustrated with reference to the following examples, which are not intended to be in any way limiting to the scope of the invention as claimed.

### Examples

### Preparatory Examples

While 3-(5-(5-Nitro-furan-2-yl)-[1,2,4]oxadiazol-3-yl)-pyridine (Compound 1) may be obtained from Ambinter Screening Library, Ambinter, Paris, France, and 3-(5-(3-Nitro-phenyl)-[1,2,4]oxadiazol-3-yl)-pyridine (Compound 2) may be obtained from ComGenex Inc., Budapest, Hungary, the following examples describe the synthesis of a number of compounds representative of the invention.

All reactions involving air sensitive reagents or intermediates were performed under nitrogen and in anhydrous solvents.

### Example 1

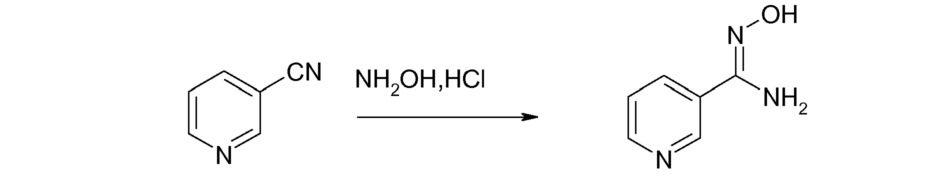

### N-Hydroxy-nicotinamidine (Intermediate compound)

Nicotinonitrile (1 g; 10 mmol) and 1.3 g of hydroxylamine hydrochloride (19 mmol) were dissolved in 15 ml of water. Sodium carbonate (2 g; 24 mmol) in 10 ml of water was continuously added, the resulting solution was stirred and heated at app. 70°C for 6 hours. Then no more starting material was left (checked by TLC), the reaction mixture was cooled to room temperature, added sodium chloride until saturation and extracted 4 times with 50 ml of ethyl acetate. The organic layer was dried with sodium sulfate and evaporated to a solid. Yield 1 g (76%) of white solid powder.

Similarly was made (Intermediate compounds):
*N*-Hydroxy-benzamidine;
*N*-Hydroxy-isonicotinamidine;
4-Fluoro-*N*-hydroxy-benzamidine;
*N*-Hydroxy-thiophene-2-carboxamidine;
*N*-Hydroxy-cyclopropane-carboxamidine;
*N*-Hydroxy-pyrazine-2-carboxamidine;
*N*-Hydroxy-2-phenyl-acetamidine;
N-Hydroxy-nicotinamidine;
N-Hydroxy-pyridine-2-carboxamidine; and
N-Hydroxy-3-nitro-benzamidine.

### Example 2

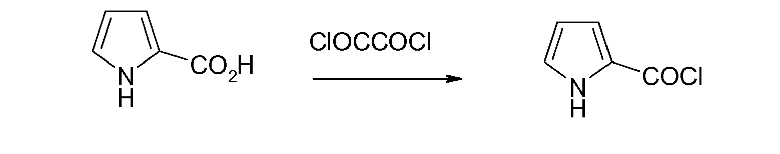

### 1H-Pyrrole-2-carbonyl-chloride (Intermediate compound)

Oxalyl chloride (6.7 g; 53 mmol) under nitrogen was cooled to 0-5°C, and 0.5 g of Pyrrole-2-carboxylic acid (4 mmol) was added. The reaction mixture was allowed to reach room temperature and heated to 50°C and stirred at this temperature, until the reaction was finished (controlled by TLC). The reaction mixture was cooled to room temperature and evaporated to an oil, the residue was washed with toluene and dried. The product was used as such in the next reaction.

Similarly was made (Intermediate compounds):
1*H*-Pyrazole-4-carbonyl chloride;
5-Nitro-furan-2-carbonyl chloride;
2-Methyl-thiazole-4-carbonyl chloride;
Benzoyl chloride;
Thiophene-2-carbonyl chloride;
3-Fluoro-benzoyl chloride;
2-Nitro-benzoyl chloride;
3-Cyano-benzoyl chloride;
4-Nitro-benzoyl chloride;
3-Chloro-benzoyl chloride;
3-Nitro-benzoyl chloride;
Thiophene-2-carbonyl chloride;
5-Bromo-thiophene-2-carbonyl chloride;
5-Bromo-furan-2-carbonyl chloride; and
6-Bromo-pyridine-2-carbonyl chloride.

### Example 3

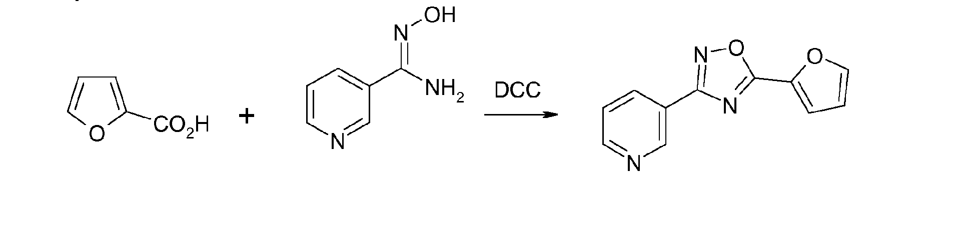

### 3-(5-Furan-2-yl-[1,2,4]oxadiazol-3-yl)-pyridine (Compound 3.1)

Furan-2-carboxylic acid (0.8 g; 7 mmol) in 15 ml of dichloromethane was cooled to 0°C, and 0.76 g of 1,3-dicyclohexylcarbodimide (4 mmol) was added slowly. The reaction mixture was stirred at 0-5°C for 2 hours and filtered. The filtrate was evaporated, the residue was dissolved in 15 ml of pyridine and added 0.43 g of N-hydroxy-nicotinamidine (3.2 mmol). The reaction mixture was heated at reflux until the reaction was finished (measured by TLC), then cooled to room temperature and poured into 100 ml of water. The precipitate was isolated by filtration and dried under vacuum. The product was isolated by column chromatrography. Yield 0.23 g (15%). Mp. 110-114°C.

Similarly was made:
3-(5-Furan-3-yl-[1,2,4]oxadiazol-3-yl)-pyridine (Compound 3.2); Mp. 105-108°C.

### Example 4

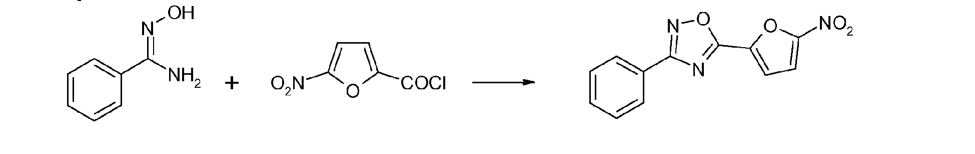

### 5-(5-Nitro-furan-2-yl)-3-phenyl-[1,2,4]oxadiazole (Compound 4.1)

N-Hydroxy-benzamidine (0.3 g; 2.1 mmol) was dissolved in 10 ml of dry pyridine and added 0.5 g of 5-nitro-furan-2-carbonyl chloride (2.8 mmol). The reaction mixture was heated at reflux for 3 hours, cooled to room temperature and poured into 50 ml of ice/water, the product precipitated out of solution and was isolated by filtration. Yield 0.3 g (41 %) of yellow solid. Mp. 164-166°C.

Similarly was made:
3-[5-(1*H*-Pyrrol-2-yl)-[1,2,4]oxadiazol-3-yl-pyridine (Compound 4.2); Mp. 200-203°C; 3-(4-Fluoro-phenyl)-5-(5-nitro-furan-2-yl)-[1,2,4]oxadiazole (Compound 4.3); Mp. 162-164°C;
3-Benzyl-5-(5-nitro-furan-2-yl)-[1,2,4]oxadiazole (Compound 4.4); Mp. 77-79°C; 5-(5-Nitro-furan-2-yl)-3-thiophen-2-yl-[1,2,4]oxadiazole (Compound 4.5); Mp. 181-185°C;
2-{5-(5-Nitro-furan-2-yl)-[1,2,4]oxadiazol-3-yl}-pyridine (Compound 4.6); Mp. 190-191°C;
2-{5-(5-Nitro-furan-2-yl)-[1,2,4]oxadiazol-3-yl}-pyrazine (Compound 4.7); Mp. 187-189°C;
3-Cyclopropyl-5-(5-nitro-furan-2-yl)-[1,2,4]oxadiazol (Compound 4.8); Mp. 67-70°C;
4-{5-(5-Nitro-furan-2-yl)-[1,2,4]oxadiazol-3-yl}-pyridine (Compound 4.9); Mp. 157-160°C;
3-{5-(1*H*-Pyrazol-4-yl)-[1,2,4]oxadiazol-3-yl}-pyridine (Compound 4.10); Mp. 219-221°C;
3-[5-(2-Methyl-thiazol-4-yl)-[1,2,4]oxadiazol-3-yl]-pyridine (Compound 4.11); Mp. 152-154°C;
3-[5-(4-Nitro-phenyl)-[1,2,4]oxadiazol-3-yl]-pyridine (Compound 4.12); Mp. 179-181°C; 2-[5-(3-Nitro-phenyl)-[1,2,4]oxadiazol-3-yl]-pyridine (Compound 4.13); 170-171°C;
3-(5-Phenyl-[1,2,4]oxadiazol-3-yl)-pyridine (Compound 4.14); Mp. 142-143°C; 3-(3-Pyridin-3-yl-[1,2,4]oxadiazol-5-yl)-benzonitrile (Compound 4.15); Mp. 154-156°C;
3-[5-(3-Chloro-phenyl)-[1,2,4]oxadiazol-3-yl]-pyridine (Compound 4.16); Mp. 122-123°C;
3-Phenyl-5-(thiophen-3-yl)-[1,2,4]oxadiazole (Compound 4.17); Mp. Mp.107-109°C; 4-[5-(3-Nitro-phenyl)-[1,2,4]oxadiazol-3-yl]-pyridine (Compound 4.18); Mp. 151-153°C;
3-[5-(3-Fluoro-phenyl)-[1,2,4]oxadiazol-3-yl]-pyridine (Compound 4.19); Mp. 112-113°C;
2-[5-(3-Nitro-phenyl)-[1,2,4]oxadiazol-3-yl]-pyrazine (Compound 4.20); Mp. 180-182°C; 3-Phenyl-5-(thiophen-2-yl)-[1,2,4]oxadiazole (Compound 4.21); Mp. 107-109°C;
3-[5-(2-Nitro-phenyl)-[1,2,4]oxadiazol-3-yl]-pyridine (Compound 4.22); Mp. 104-105°C; 3-[5-(3-Trifluoromethyl-phenyl)-[1,2,4]oxadiazol-3-yl]-pyridine (Compound 4.23); Mp. 78-83°C;
3-[3-(3-Nitro-phenyl)-[1,2,4]oxadiazol-5-yl]-pyridine (Compound 4.24); Mp. 173-175°C; N-[3-(3-Pyridin-3-yl-[1,2,4]oxadiazol-5-yl)-phenyl]-acetamide (Intermediate);
2-Bromo-6-(3-pyridin-3-yl-[1,2,4]oxadiazol-5-yl)-pyridine (Intermediate); 3-[5-(5-Bromo-furan-2-yl)-[1,2,4]oxadiazol-3-yl]-pyridine (Intermediate); and 3-[5-(5-Bromo-thiophen-2-yl)-[1,2,4]oxadiazol-3-yl]-pyridine (Intermediate).

### Example 5

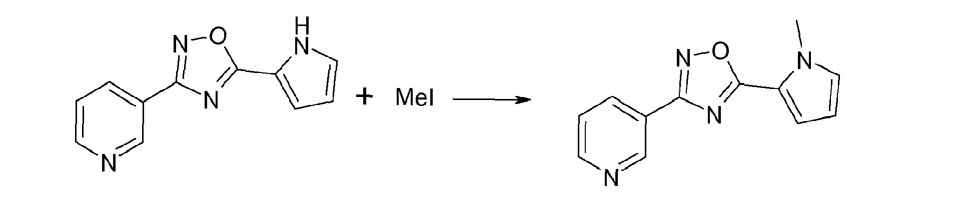

### 3-{5-(1-Methyl-1H-pyrrol-2-yl)-[1,2,4]oxadiazol-3-yl}-pyridine (Compound 5.1)

3-{5-(1*H*-pyrrol-2-yl)-[1,2,4]oxadiazol-3-yl]-pyridine (1 g; 0.5 mmol) in 15 ml of dry THF at -70°C was added 0.18 g of sodium hexamethyl disilazide (1 mmol), the reaction mixture was stirred at -70°C for 30 min. and at 0°C for 1 hour. The reaction mixture was cooled to -70°C and added 0.076 g of iodomethane (0.52 mmol). The reaction mixture was stirred at -70°C for ½ hour, then at room temperature overnight. The product was isolated by column chromatography. Yield 0.04 g of yellow solid (37%). Mp. 108-109°C.

### Example 6

### 6-(Pyridin-3-yl-[1,2,4]oxadiazol-5-yl)-pyridine-2-carbonitrile (Compound 6.1)

2-Bromo-6-(3-pyridin-3-yl-[1,2,4]oxadiazol-5-yl)-pyridine (250 mg, 0.83 mmol) and 80 mg of potassium cyanide (1.24 mmol) in 15 ml of acetonitrile was degassed three times (vacuum/nitrogene), added a solution of 24 µl Tributyltin chloride (1 µmol) in heptane, 2.3 mg of bis-(diphenylphosphino)ferrocene (4.1 µmol) and 4 mg of bispalladium tris(dibenzylidene acetone) (4.1 µmol) were added. The suspension was degassed three times and stirred at ambident temperature for 30 minutes. The mixture was degassed again and heated at 80°C for 17 hours. The reaction mixture concentrated, residue was diluted with ethyl acetate and washed with water. The organic layer was dried with sodium sulphate, concentrated, and purified by column chromatography over silica gel using 20% ethyl acetate in petroleum ether, Yield 80 mg. Mp 201-203°C.

Similarly was made:
5-(3-Pyridin-3-yl-[1,2,4]oxadiazol-5-yl)-furan-2-carbonitrile (Compound 6.2); Mp. 141-144°C; and
5-(3-Pyridne-3-yl-[1.2.4]oxadiazol-5-yl)-thiophene-2-carbonitrile (Compound 6.3); Mp. 159-161 °C.

### Example 7

### 3-(3-Pyridin-3-yl-[1,2,4]oxadiazol-5-yl)-phenylamine (Compound 7.1)

To a saturated solution of hydrogen chloride in ehthanol (20 ml) at 0°C, was added 0.48 g of N-[3-pyridin-3-yl[1,2,4]oxadiazol-5-yl)-phenyl]-acetamide (1.7 mmol) portion wise, after addition, the reaction mixture was allowed to reach room temperature and heated at 50°C for 15 hours. The reaction mixture was evaporated to an oil and added water. The mixture was added saturated sodium bicarbonate (aq.) and extracted with ethyl acetate, the organic phase was washed with brine, dried with sodium sulphate and evaporated to an oil. The product was isolated by column chromatography. Yield 0.2 g (48%). Mp.161-163°C.

### Example 8

### Biological Activity

This experiment illustrates the biological activity of a combination of substances according to the invention.

As positive allosteric modulators of nicotine receptors 3-(3-Pyridin-3-yl-[1,2,4]oxadiazol-5-yl)-benzonitrile (Compound 4.15) was investigated.

As cognition enhancer 1-(3-Pyridyl)-homopiperazine (Compound 1 F of WO 99/21834) was investigated.

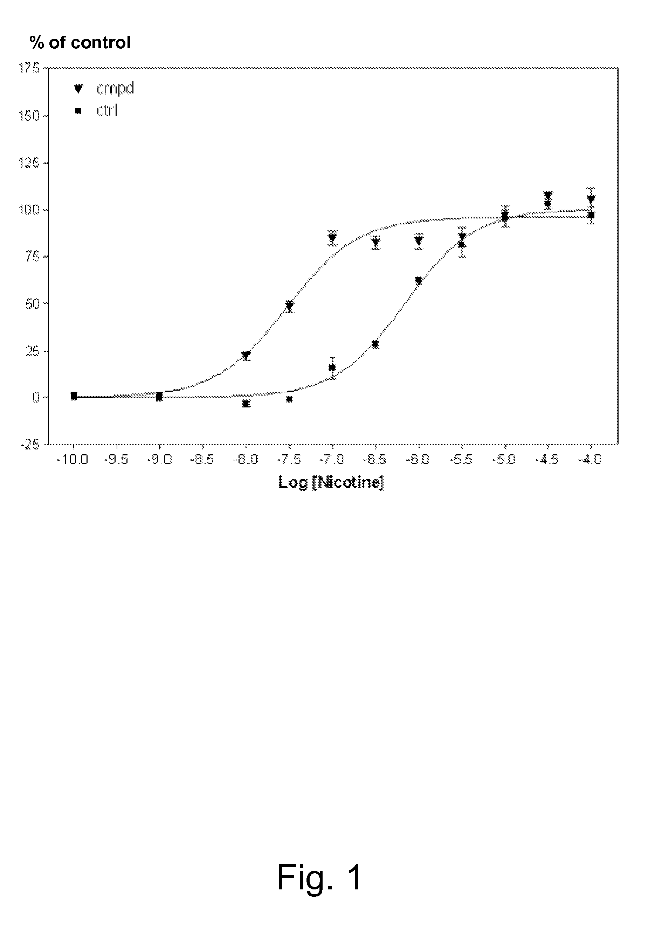

In a Fluorometric Laser Imaging Plate Reader (FLIPR) system a 10-100 fold left-shift of the (-)nicotine dose-response curve was demonstrated in the presence of 1-10 µM of each of the positive allosteric modulators (Compound 4.15 and Compound 6.3). The effects of using Compound 4.15 as positive allosteric modulator are presented in Fig. 1.

In a mouse marble burying paradigm the inventive combination of the positive allosteric modulator, Compound 4.15, and Compound 1 F of WO 99/21834 as nicotine acetylcholine receptor agonist, demonstrated a 6-10 fold left-shift of the dose-responce curve relative to the nicotine acetylcholine receptor agonist alone. Doses of the positive allosteric modulator (Compound 4.15) alone were found inactive in this paradigm.

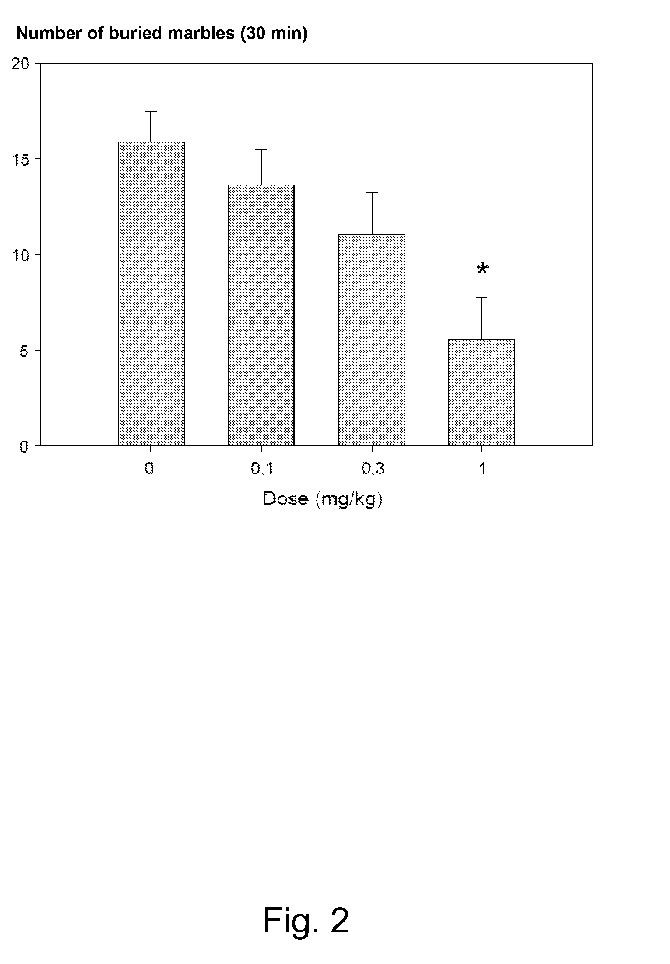

The results of these experiments are shown in Figs. 2-3.

### The following clauses represent special embodiments of the invention:

Clause 1. A pharmaceutical composition comprising a therapeutically effective amount of
   (i) a positive allosteric modulator of nicotine receptors; and
   (ii) a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug;
      or pharmaceutically-acceptable addition salts thereof, together with one or more adjuvants, excipients, carriers and/or diluents.
Clause 2. The pharmaceutical composition of clause 1, wherein the positive allosteric modulator of nicotine receptors is a nicotine α₇ receptor modulator.
Clause 3. The pharmaceutical composition of clause 2, wherein the positive allosteric modulator of the nicotine α₇ receptor is a urea derivative selected from the group consisting of
   *N*-(3-Chloro-6-hydroxy-phenyl)-*N*'-(2-chloro-5-trifluoromethyl-phenyl)-urea;
   *N*-(2-Amino-6-hydroxy-phenyl)-*N'*-(3-trifluoromethyl-phenyl)-urea;
   *N*-(5-Chloro-2-hydroxy-phenyl)-*N'*-(2-hydroxy-4-nitro-phenyl)-urea;
   *N*-(2-Amino-4,5-dichloro-phenyl)-*N'*-(2-chloro-5-trifluoromethyl-phenyl)-urea;
   *N*-{4,5-Dichloro-2-[3-(2-chloro-5-trifluoromethyl-phenyl)-ureido]phenyl}-acetamide;
   *N*-(3-Chloro-4-hydroxy-phenyl)-*N'*-(3-trifluoromethyl-phenyl)-urea;
   *N*-(4-Hydroxy-6-methyl-phenyl)-*N'*-(3-trifluoromethyl-phenyl)-urea;
   *N*-(3,5-Dichloro-4-hydroxy-phenyl)*-N'*-(3-trifluoromethyl-phenyl) urea;
   *N*-(2-Chloro-5-trifluoromethyl-phenyl)-*N'*-(3,5-dichloro-4-hydroxy-phenyl) urea;
   *N*-(Biphenyl-3-yl)-*N'*-(3,5-dichloro-4-hydroxy-phenyl) urea;
   *N*-(Biphenyl-4-yl)-*N'*-(3,5-dichloro-4-hydroxy-phenyl) urea;
   *N*-(Biphenyl-4-yl)-*N'*-(5-chloro-2-hydroxy-phenyl) urea;
   *N*-(3,5-Dichloro-2-hydroxy-phenyl)-*N'*-(2-chloro-5-trifluoromethyl-phenyl)-urea;
   *N*-(3-Bromo-5-chloro-2-hydroxy-phenyl)-*N'*-(2-chloro-5-trifluoromethylphenyl)-urea;
   *N*-(2-Chloro-5-trifluoromethyl-phenyl)-*N'*-(3-hydroxy-5-methyl-phenyl) urea;
   *N*-(3-Hydroxy-5-methyl-phenyl)-*N'*-(3-trifluoromethyl-phenyl) urea;
   *N*-(2-Chloro-5-trifluoromethyl-phenyl)-*N'*-(4-hydroxy-2-methyl-phenyl) urea;
   *N*-(5-Chloro-2-methoxy-phenyl)-*N'*-(2-chloro-5-trifluoromethyl-phenyl) urea;
   *N*-(2-Hydroxy-6-nitro-phenyl)-*N'*-(3-trifluoromethyl-phenyl) urea; and
   *N*-(3-Chloro-6-methoxy-phenyl)-*N'*-(2-hydroxy-4-nitro-phenyl) urea;
      or an enantiomer or a mixture of its enantiomers, or a pharmaceutically-acceptable addition salt thereof.
Clause 4. The pharmaceutical composition of clause 3, wherein the positive allosteric modulator of the nicotine α₇ receptor is
   *N*-(3-Chloro-6-hydroxy-phenyl)-*N'*-(2-chloro-5-trifluoromethyl-phenyl)-urea;
   or an enantiomer or a mixture of its enantiomers, or a pharmaceutically-acceptable addition salt thereof.
Clause 5. The pharmaceutical composition of clause 1, wherein the positive allosteric modulator of nicotine receptors is a nicotine α₄β₂ receptor modulator.
Clause 6. The pharmaceutical composition of clause 5, wherein the positive allosteric modulator of the nicotine α₄β₂ receptors is an oxadiazole derivative selected from the group consisting of
   3-Cyclopropyl-5-(5-nitro-furan-2-yl)-[1,2,4]oxadiazole;
   5-(5-Nitro-furan-2-yl)-3-phenyl-[1,2,4]oxadiazole;
   5-(5-Nitro-furan-2-yl)-3-(4-fluoro)-phenyl-[1,2,4]oxadiazole;
   5-(5-Nitro-furan-2-yl)-3-benzyl-[1,2,4]oxadiazole;
   5-(5-Nitro-furan-2-yl)-3-thiophen-2-yl-[1,2,4]oxadiazole;
   2-(5-(5-Nitro-furan-3-yl)-[1,2,4]oxadiazol-3-yl)-pyridine;
   3-(5-(3-Nitro-phenyl)-[1,2,4]oxadiazol-3-yl)-pyridine;
   3-(5-Furan-2-yl-[1,2,4]oxadiazol-3-yl)-pyridine;
   3-(5-(5-Nitro-furan-3-yl)-[1,2,4]oxadiazol-3-yl)-pyridine;
   3-(5-Furan-3-yl-[1,2,4]oxadiazol-3-yl)-pyridine;
   3-[5-(1H-Pyrrol-2-yl)-[1,2,4]oxadiazol-3-yl]-pyridine;
   4-(5-Furan-2-yl-[1,2,4]oxadiazol-3-yl)-pyridine;
   2-[5-(5-Nitro-furan-2-yl)-[1,2,4]oxadiazol-3-yl]-pyrazine;
   3-[5-(1-Methyl-1H-pyrrol-2-yl)-[1,2,4]oxadiazol-3-yl]-pyridine;
   3-[5-(1H-Pyrazol-4-yl)-[1,2,4]oxadiazol-3-yl]-pyridine;
   3-[5-(2-Methyl-thiazol-4-yl)-[1,2,4]oxadiazol-3-yl]-pyridine;
   3-[5-(4-Nitro-phenyl)-[1,2,4]oxadiazol-3-yl]-pyridine;
   2-[5-(3-Nitro-phenyl)-[1,2,4]oxadiazol-3-yl]-pyridine;
   3-(5-Phenyl-[1,2,4]oxadiazol-3-yl)-pyridine;
   3-(3-Pyridin-3-yl-[1,2,4]oxadiazol-5-yl)-benzonitrile;
   3-[5-(3-Chloro-phenyl)-[1,2,4]oxadiazol-3-yl]-pyridine;
   3-Phenyl-5-(thiophen-3-yl)-[1,2,4]oxadiazole;
   4-[5-(3-Nitro-phenyl)-[1,2,4]oxadiazol-3-yl]-pyridine;
   3-[5-(3-Fluoro-phenyl)-[1,2,4]oxadiazol-3-yl]-pyridine;
   2-[5-(3-Nitro-phenyl)-[1,2,4]oxadiazol-3-yl]-pyrazine;
   3-Phenyl-5-(thiophen-2-yl)-[1,2,4]oxadiazole;
   3-[5-(2-Nitro-phenyl)-[1,2,4]oxadiazol-3-yl]-pyridine;
   3-[5-(3-Trifluoromethyl-phenyl)-[1,2,4]oxadiazol-3-yl]-pyridine;
   3-[3-(3-Nitro-phenyl)-[1,2,4]oxadiazol-5-yl]-pyridine;
   6-(Pyridin-3-yl-[1,2,4]oxadiazol-5-yl)-pyridine-2-carbonitrile;
   5-(3-Pyridin-3-yl-[1,2,4]oxadiazol-5-yl)-furan-2-carbonitrile;
   5-(3-Pyridin-3-yl-[1.2.4]oxadiazol-5-yl)-thiophene-2-carbonitrile; and 3-(3-Pyridin-3-yl-[1,2,4]oxadiazol-5-yl)-phenylamine;
   any of its isomers or any mixture of isomers, or a pharmaceutically-acceptable addition salt thereof.
Clause 7. The oxadiazole derivative of clause 6, which is 3-(3-Pyridin-3-yl-[1,2,4]oxadiazol-5-yl)-benzonitrile;
   any of its isomers or any mixture of isomers, or a pharmaceutically-acceptable addition salt thereof.
Clause 8. The pharmaceutical composition of any one of clauses 1-7, wherein the cognitive enhancer is an nicotine acetylcholine receptor agonist selected from the group consisting of Nicotine, ABT-594, SSR-180711A, PNU-282987, AR-R17779, Varenicline, Isopronicline (TC-1734), 1-(3-Pyridyl)-homopiperazine and 1-(3-Chloro-5-pyridyl)homopiperazine, any of its isomers or any mixture of isomers, or a pharmaceutically-acceptable addition salt thereof.
Clause 9. The pharmaceutical composition of any one of clauses 1-7, wherein the cognitive enhancer is an acetylcholine esterase inhibitor selected from the group consisting of Tacrin, Donepezil, Rivastigmine and Galantamine, any of its isomers or any mixture of isomers, or a pharmaceutically-acceptable addition salt thereof.
Clause 10. The pharmaceutical composition of any one of clauses 1-7, wherein the cognitive enhancer is a positive AMPA receptor modulator selected from the group consisting of CX-516, CX-614, Cyclothiazid, Piracetam and Aniracetam, any of its isomers or any mixture of isomers, or a pharmaceutically-acceptable addition salt thereof.
Clause 11. The pharmaceutical composition of any one of clauses 1-7, wherein the cognitive enhancer is an antipsychotic drug selected from the group consisting of Haloperidol, Fluphenazine, Chlorpromazine, Pimozide, Clozapine, Olanzapine, Ziprasidone, Risperidone, Quetiapine, Aripiprazole, Sertindole, Zotepine and Amisulpride.
Clause 12. The pharmaceutical composition of any one of clauses 1-7, wherein the cognitive enhancer is an antidepressant drug selected from the group consisting of Bupropion, Citalopram, Desipramine, Duloxetine, Escitalopram, Fenfluramine, Fluoxetine, Fluvoxamine, Imipramine, Paroxetine, Radafaxine, Sibutramine, Sertraline and Venlafaxine.
Clause 13. The pharmaceutical composition of any one of clauses 1-7, wherein the cognitive enhancer is an anti Parkinson drug selected from the group consisting of Nomifensine, Bromocriptine, Levodopa, Pergolide, Pramipexole and Ropinerole.
Clause 14. Use of a combination of
   (i) a positive allosteric modulator of nicotine receptors; and
   (ii) a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug;
      or pharmaceutically-acceptable addition salts thereof,
      for the manufacture of a medicament for the treatment, prevention or alleviation of a cognitive dysfunction of a mammal, including a human.
Clause 15. The use according to clause 14, wherein the cognitive dysfunction is a disorder or condition selected from the group consisting of Alzheimer's Disease (AD), mild cognitive impairment, age-related cognitive decline, vascular dementia, Parkinson's disease, Huntington's disease, memory impairment associated with depresssion or anxiety, schizophrenia, Down's syndrome, stroke, traumatic brain injury (TBI), AIDS associated dementia and attention deficit disorder.
Clause 16. A kit of parts comprising at least two separate unit dosage forms (A) and (B):
   (A) a positive allosteric modulator of nicotine receptors; and
   (B) a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug; and optionally
   (C) instructions for the simultaneous, sequential or separate administration of the positive allosteric nicotine receptor modulator of (A) and the cognitive enhancer of (B) to a patient in need thereof.
Clause 17. A method of treatment, prevention or alleviation of a cognitive dysfunction of a living animal body, including a human, which method comprises the step of administering to such a living animal body in need thereof, a therapeutically effective amount of a combination of
   (i) a positive allosteric modulator of nicotine receptors; and
   (ii) a cognitive enhancer selected from the group consisting of a nicotine acetylcholine receptor agonist, an acetylcholine esterase inhibitor, a positive AMPA receptor modulator, an antipsychotic drug, an antidepressant drug and an anti Parkinson drug;
      or pharmaceutically-acceptable addition salts thereof.

## Claims

1. A pharmaceutical composition comprising a therapeutically effective amount of
(i) a positive allosteric modulator of nicotine receptors which modulator is a nicotine α₄β₂ receptor modulator; and
(ii) a nicotine acetylcholine receptor agonist;
or pharmaceutically-acceptable addition salts thereof, together with one or more adjuvants, excipients, carriers and/or diluents.

2. The pharmaceutical composition of claim 1, wherein the positive allosteric modulator of the nicotine α₄β₂ receptors is an oxadiazole derivative selected from the group consisting of
3-Cyclopropyl-5-(5-nitro-furan-2-yl)-[1,2,4]oxadiazole;
5-(5-Nitro-furan-2-yl)-3-phenyl-[1,2,4]oxadiazole;
5-(5-Nitro-furan-2-yl)-3-(4-fluoro)-phenyl-[1,2,4]oxadiazole;
5-(5-Nitro-furan-2-yl)-3-benzyl-[1,2,4]oxadiazole;
5-(5-Nitro-furan-2-yl)-3-thiophen-2-yl-[1,2,4]oxadiazole;
2-(5-(5-Nitro-furan-3-yl)-[1,2,4]oxadiazol-3-yl)-pyridine;
3-(5-(3-Nitro-phenyl)-[1,2,4]oxadiazol-3-yl)-pyridine;
3-(5-Furan-2-yl-[1,2,4]oxadiazol-3-yl)-pyridine;
3-(5-(5-Nitro-furan-3-yl)-[1,2,4]oxadiazol-3-yl)-pyridine;
3-(5-Furan-3-yl-[1,2,4]oxadiazol-3-yl)-pyridine;
3-[5-(1H-Pyrrol-2-yl)-[1,2,4]oxadiazol-3-yl]-pyridine;
4-(5-Furan-2-yl-[1,2,4]oxadiazol-3-yl)-pyridine;
2-[5-(5-Nitro-furan-2-yl)-[1,2,4]oxadiazol-3-yl]-pyrazine;
3-[5-(1-Methyl-1H-pyrrol-2-yl)-[1,2,4]oxadiazol-3-yl]-pyridine;
3-[5-(1H-Pyrazol-4-yl)-[1,2,4]oxadiazol-3-yl]-pyridine;
3-[5-(2-Methyl-thiazol-4-yl)-[1,2,4]oxadiazol-3-yl]-pyridine;
3-[5-(4-Nitro-phenyl)-[1,2,4]oxadiazol-3-yl]-pyridine;
2-[5-(3-Nitro-phenyl)-[1,2,4]oxadiazol-3-yl]-pyridine;
3-(5-Phenyl-[1,2,4]oxadiazol-3-yl)-pyridine;
3-(3-Pyridin-3-yl-[1,2,4]oxadiazol-5-yl)-benzonitrile;
3-[5-(3-Chloro-phenyl)-[1,2,4]oxadiazol-3-yl]-pyridine;
3-Phenyl-5-(thiophen-3-yl)-[1,2,4]oxadiazole;
4-[5-(3-Nitro-phenyl)-[1,2,4]oxadiazol-3-yl]-pyridine;
3-[5-(3-Fluoro-phenyl)-[1,2,4]oxadiazol-3-yl]-pyridine;
2-[5-(3-Nitro-phenyl)-[1,2,4]oxadiazol-3-yl]-pyrazine;
3-Phenyl-5-(thiophen-2-yl)-[1,2,4]oxadiazole;
3-[5-(2-Nitro-phenyl)-[1,2,4]oxadiazol-3-yl]-pyrid ine;
3-[5-(3-Trifluoromethyl-phenyl)-[1,2,4]oxadiazol-3-yl]-pyridine;
3-[3-(3-Nitro-phenyl)-[1,2,4]oxadiazol-5-yl]-pyrid ine;
6-(Pyridin-3-yl-[1,2,4]oxadiazol-5-yl)-pyridine-2-carbonitrile;
5-(3-Pyridin-3-yl-[1,2,4]oxadiazol-5-yl)-furan-2-carbonitrile;
5-(3-Pyridin-3-yl-[1.2.4]oxadiazol-5-yl)-thiophene-2-carbonitrile; and
3-(3-Pyridin-3-yl-[1,2,4]oxadiazol-5-yl)-phenylamine;
any of its isomers or any mixture of isomers, or a pharmaceutically-acceptable addition salt thereof.

3. The oxadiazole derivative of claim 2, which is
3-(3-Pyridin-3-yl-[1,2,4]oxadiazol-5-yl)-benzonitrile;
any of its isomers or any mixture of isomers, or a pharmaceutically-acceptable addition salt thereof.

4. The pharmaceutical composition of any one of claims 1-3, wherein the nicotine acetylcholine receptor agonist is selected from the group consisting of Nicotine, ABT-594, SSR-180711A, PNU-282987, AR-R17779, Varenicline, Isopronicline (TC-1734), 1-(3-Pyridyl)-homopiperazine and 1-(3-Chloro-5-pyridyl)homopiperazine, any of its isomers or any mixture of isomers, or a pharmaceutically-acceptable addition salt thereof.

5. Use of a combination of
(i) a positive allosteric modulator of nicotine receptors which modulator is a nicotine α₄β₂ receptor modulator; and
(ii) a nicotine acetylcholine receptor agonist;
or pharmaceutically-acceptable addition salts thereof,
wherein the positive allosteric modulator of nicotine receptors is a nicotine α₄β₂ receptor modulator, and
wherein the cognitive enhancer is an nicotine acetylcholine receptor agonist for the manufacture of a medicament for the treatment, prevention or alleviation of a cognitive dysfunction of a mammal, including a human.

6. The use according to claim 5, wherein the cognitive dysfunction is a disorder or condition selected from the group consisting of Alzheimer's Disease (AD), mild cognitive impairment, age-related cognitive decline, vascular dementia, Parkinson's disease, Huntington's disease, memory impairment associated with depresssion or anxiety, schizophrenia, Down's syndrome, stroke, traumatic brain injury (TBI), AIDS associated dementia and attention deficit disorder.

7. A kit of parts comprising at least two separate unit dosage forms (A) and (B):
(A) a positive allosteric modulator of nicotine receptors which modulator is a nicotine α₄β₂ receptor modulator; and
(B) a nicotine acetylcholine receptor agonist; and optionally
(C) instructions for the simultaneous, sequential or separate administration of the positive allosteric nicotine receptor modulator of (A) and the nicotine acetylcholine receptor agonist of (B) to a patient in need thereof.

8. A combination of
(i) a positive allosteric modulator of nicotine receptors which modulator is a nicotine α₄β₂ receptor modulator; and
(ii) a nicotine acetylcholine receptor agonist;
or pharmaceutically-acceptable addition salts thereof,
for use as a medicament.

9. A combination of
(i) a positive allosteric modulator of nicotine receptors which modulator is a nicotine α₄β₂ receptor modulator; and
(ii) a nicotine acetylcholine receptor agonist;
or pharmaceutically-acceptable addition salts thereof,
for use as a medicament for the treatment, prevention or alleviation of a cognitive dysfunction of a mammal, including a human.
